Europäisches Patentamt

European Patent Office

Office européen des brevets

(19)

(11) Publication number: **0 400 904**
**A1**

## (12) EUROPEAN PATENT APPLICATION

(21) Application number: **90305686.9**

(22) Date of filing: **24.05.90**

(51) Int. Cl.⁵: **C07C 67/00, C07C 69/14, C07D 307/33, B01J 23/68**

(30) Priority: **27.05.89 GB 8917157**

(43) Date of publication of application:
**05.12.90 Bulletin 90/49**

(84) Designated Contracting States:
**AT BE DE ES FR GB IT LU NL SE**

(71) Applicant: **BP Chemicals Limited**
**Belgrave House 76 Buckingham Palace Road**
**London, SW1W 0SU(GB)**

Applicant: **The British Petroleum Company**
**p.l.c.**
**Britannic House Moor Lane**
**London EC2Y 9BU(GB)**

(72) Inventor: **McLachlan, Kenneth Alan**
**BP Chemicals Limited, Salt End**
**Hedon, Hull, HU12 8DS(GB)**
Inventor: **Williams, Peter Sefton**
**BP Chemicals Limited, Salt End**
**Hedon, Hull, HU12 8DS(GB)**

(74) Representative: **Barlow, Michael Thomas et al**
**BP INTERNATIONAL LIMITED Patents &**
**Agreements Division Chertsey Road**
**Sunbury-on-Thames Middlesex TW16**
**7LN(GB)**

(54) **Ester production by hydrogenation of carboxylic acids and anhydrides.**

(57) Carboxylic acid esters are produced from the corresponding $C_2$ to $C_{12}$ carboxylic acids or anhydrides by hydrogenation at elevated temperature, in the presence of a catalyst comprising (a) an alloy of a Group VIII noble metal and (b) an oxide of a Group IVb element, preferably titania. A preferred catalyst comprises palladium/silver alloy optionally with rhenium. The process is suitable for hydrogenating acetic acid to ethyl acetate and maleic acid to gamma-butyrolactone.

EP 0 400 904 A1

## ESTER PRODUCTION BY HYDROGENATION OF CARBOXYLIC ACIDS AND ANNYDRIDES

The present invention relates to the hydrogenation of carboxylic acids and their anhydrides.

The hydrogenation of carboxylic acids to produce alcohols has long been known. Representative of the prior art may be mentioned US Patents Nos 4,446,073; 4,398,039; 4,104.478; 3,985,814 and 4,524,255; DE-A-2,605,107, GB-A-1534232 and GB-A-1551741.

US Patent No. 4,446,073 discloses the reduction of unsaturated carboxylic acids to esters or alcohols using a catalyst comprising low valence oxidation state cadmium and ruthenium and optionally a platinum group metal composited on a solid support.

US Patent No. 4,393,039 discloses the vapour phase hydrogenation of carboxylic acids to the corresponding alcohols in the presence of steam and a catalyst comprising the mixed oxides of ruthenium and at least one of cobalt, nickel, and optionally one of cadmium, zinc, copper, iron, rhodium, palladium, osmium, iridium and platinum. The catalyst may be supported on an inert carrier such as aluminates, silicates, titanates, zirconia and mixtures thereof. In the absence of steam carboxylic acid esters were also prepared.

US Patent No. 4,104,478 discloses the conversion of fatty acids into the corresponding fatty alcohols by hydrogentation over catalysts containing rhenium and ruthenium, rhodium, palladium or platinum.

US Patent No. 3,985,814 discloses a process for converting $-CO_2H$ groups to $-CH_2OH$ groups by hydrogenation using a platinum catalyst.

US Patent No. 4,524,225 discloses the hydrogenation of fatty acids using as catalyst combinations of copper, chromium, ruthenium, platinum, palladium and rhenium supported on alpha-alumina, theta-alumina, titanated alumina, titania or aluminium phosphate.

DE-A-2,605,107 discloses the hydrogenation of carboxylic acids to alcohols catalysed by palladium/rhenium combinations.

GB-A-1534232 discloses a process for the production of an alcohol having five or more carbon atoms by the catalytic hydrogenation of the corresponding carboxylic acid or of a lactone or anhydride thereof in the presence of water and/or other solvents and a catalyst comprising palladium and rhenium and an acid-resistant carrier, eg silica, alumina or activated charcoal.

GB-A-1551741 discloses a process for preparing butanediol(1,4) by hydrogenating maleic anhydride, maleic acid or a mixture thereof in the presence of a catalyst comprising an element of Group VIIA of the Mendeleef Periodic Table or a compound thereof and ruthenium, rhodium, palladium, osmium, iridium or platinum or a compound thereof or a mixture of such elements and compounds.

More recent patent publications include our EP-A-0198681 relating to the production of either ethanol from acetic acid or propanol from propionic acid in the vapour phase by contact with hydrogen and a catalyst comprising (i) a noble metal of Group VIII of the Periodic Table and (ii) rhenium, optionally supported on a high surface area graphitised carbon, a graphite, a silica, an alumina or a silica/alumina; EP-A-0198682 relating to the production of either an alcohol and/or a carboxylic acid ester from a $C_2$ to $C_{12}$ carboxylic acid by contact with hydrogen and a heterogeneous catalyst comprising (i) molybdenum or tungsten and (ii) a noble metal of Group VIII of the Periodic Table, optionally supported on a high surface area graphitised carbon, a graphite, a silica, and alumina or a silica/alumina; EP-A-0 210 795 relating to the production of an alcohol by hydrogenating a carboxylic acid in the presence of a reductively activated solid copper-containing catalyst additionally incorporating at least one of magnesium, a lanthanide metal or an actinide metal and EP-A-0 285 420 relating to the production from either a carboxylic acid or an anhydride thereof of the corresponding alcohol and/or carboxylic acid ester by reaction with hydrogen in the presence as catalyst of a composition comprising an alloy of (i) at least one noble metal of Group VIII of the Periodic Table and (ii) at least one metal capable of alloying with the noble metal, for example silver, optionally supported on a high surface area graphitised carbon, a graphite, an activated carbon, a silica, an alumina or a silica/alumina.

There remains a need for a process in which $C_2$ to $C_{12}$ carboxylic acids and their anhydrides can be hydrogenated preferentially to their corresponding carboxylic acid esters.

Accordingly, the present invention provides a process for the production from a $C_2$ to $C_{12}$ carboxylic acid or an anhydride thereof of the corresponding carboxylic acid ester which process comprises reacting at elevated temperature the acid or anhydride with hydrogen in the presence as catalyst of a composition comprising (a) an alloy of (i) at least one noble metal of Group VIII of the Periodic Table of the Elements, and (ii) at least one metal capable of alloying with the aforesaid Group VIII noble metal, and (b) an oxide of an element of Group IVb of the Periodic Table of the Elements.

The Periodic Table of the Elements referred to in this specification is that to be found in the Handbook of Chemistry and Physics, 63rd edition.

It is believed, though the belief is not to be interpreted as binding in any manner, that the Group IVB-containing catalyst of the present invention behave quite differently to the carbon and silica supported catalysts of, for example EP-A-0 198 681, in that the latter catalysts produce an alcohol as the first-formed product and the ester as the second-formed product arises from the reaction of the alcohol with unreacted carboxylic acid or anhydride. The ratio of ester to alcohol is limited in this case by the thermodynamic equilibrium of the esterification reaction, typically in the case of acetic acid hydrogenation to an ethyl acetate to ethanol ratio of about 1.9:1. In contrast, the catalyst of the present invention is believed to form the ester as the first-formed product and thereafter, at high acid conversions, it is believed that the ester is hydrogenated to the alcohol. Whether this theory be true or not, it is nevertheless observed that at low acid conversion the formation of alcohol is at a low level. Thus, working at low conversions, it is possible to produce from acetic acid, for example, ethyl acetate at a selectivity of about 90% with accompanying ethanol selectivity of about 10%. It may even be possible to eliminate alcohol formation completely. The capability of the process of the invention to produce an ester free from alcohol would provide the advantage that the generally difficult separation of the alcohol from the ester can be avoided. Moreover, the selective production of esters from carboxylic acids is a desirable objective in its own right because it avoids the requirement for an alcohol, which is reacted with an acid in conventional esterification processes for producing esters.

The process of the invention is applicable to both $C_2$ to $C_{12}$ carboxylic acids and their anhydrides. The carboxylic acid or anhydride thereof may be either saturated or unsaturated. Mono-, di- or polybasic carboxylic acids and their anhydride derivatives may be employed. Suitable monobasic acids include acids having the formula R-COOH, wherein R is a substituted or unsubstituted aliphatic or aromatic group, which acids are hydrogenated to esters of the formula RCOOR. Suitably the group R may be a $C_1$ to $C_{12}$, typically a $C_1$ to $C_5$ alkyl group. Examples of suitable monobasic acids include acetic acid, propionic acid, butyric acids and heptanoic acids. Preferred monobasic acids include acetic acid from which there is obtained ethyl acetate and propionic acid from which there is obtained propyl propionate.

Alternatively, dibasic acids, polybasic acids and their anhydrides may be employed. Suitable dibasic acids may be both saturated or unsaturated. Examples of suitable dibasic acids include glutaric acid, glutaric anhydride, adipic acid, adipic anhydride, succinic acid, succinic anhydride, maleic acid and maleic anhydride. Preferred are $C_4$ dibasic acids and their anhydrides. In the case of maleic acid the main ester product is the internal ester gamma butyrolactone. Mixtures of acids, anhydrides and acids/anhydrides may also be employed.

Hydrogen is commercially available on a large scale and may be used with or without further purification. A desirable purification may be removal of carbon dioxide.

As regards the catalyst composition, the noble metals of Group VIII of the Periodic Table of the Elements are palladium, platinum, rhodium, ruthenium, osmium and iridium. Of the aforesaid noble metals, palladium, rhodium and ruthenium are preferred. Metals capable of alloying with palladium include silver, gold, copper, nickel, rhodium, tin, cobalt, aluminium, manganese, gallium, iron, chromium and platinum, of which silver, gold and copper are preferred, silver being more preferred. Metals capable of alloying with ruthenium include iron, cobalt, manganese, germanium and rhenium.

For the purpose of this specification at least the elements of Group IVb of the Periodic Table of the Elements are titanium, zirconium and hafnium. The elements of Group IVb are present in the catalyst in the form of their oxides. A preferred oxide is titania. Titania exists in the forms of anatase titania and rutile titania. There are indications that anatase titania or a mixture of anatase and rutile titania principally comprising anatase titania may be preferred.

The catalyst composition may additionally incorporate rhenium.

The catalyst may be used in the form of the Group VIII noble metal alloy (a) and optionally rhenium, supported on the oxide of the Group IVb element (b) or in the form of component (a), optionally rhenium together with component (b), supported on an inert support. Suitable inert supports include high surface area graphitised carbons (HSAGs), graphites, activated carbons, silicas, aluminas and silica/aluminas.

The catalyst may incorporate one or more further components. Thus, one or more metals selected from Groups IA or Group IIA of the Periodic Table of the Elements may be incorporated. A suitable metal is potassium.

Suitably the catalyst comprises from 0.1 to 20%, preferably from 1 to 10%, by weight of component (a) and optionally from 0.1 to 20%, preferably from 1 to 10%, weight of rhenium.

Examples of suitable catalysts include palladium - silver alloy/titania and palladium - silver alloy/rhenium/titania.

The alloy constituting component (a) of the catalyst composition may be produced in any suitable manner, for example thermally or by a colloidal method. The component (a) may then be admixed with component (b).

The catalysts for use in the process of the invention may suitably be prepared by impregnation. One method of preparing the catalyst comprises impregnating component (b) with a solution of soluble thermally decomposable compounds of component (a) and thereafter thermally decomposing the thermally decomposable compounds to the metal and/or metal oxide and forming an alloy thereof.

Another method of preparing the catalyst comprises contacting component (b) with an aqueous solution of compounds of component (a), precipitating the compounds onto component (b), by for example addition of sodium disilicate and then reducing the compounds of component (a), by for example, addition of hydrazine, followed by evaporation of the solvent. The alloy impregnated component (b) may be subsequently impregnated with a rhenium compound for example. This coprecipitation method of alloy formation avoids the risks of damaging component (b), associated with a thermal method.

Before use in the process of the invention the catalyst is preferably activated by contact at elevated temperature with hydrogen, optionally also containing an inert gas, for example nitrogen, for a suitable period, for example from 1 to 24 hours. Activation may be accomplished by the catalyst manufacturer or the process operator immediately prior to operation of the process or both.

As regards the process for producing the ester, this may be operated either in the liquid phase or the vapour phase. It may be operated either batchwise or continuously. Continuous operation is preferred, with recycle of unconverted carboxylic acid or anhydride being a desirable option. The catalyst may be employed in the form of a fixed bed, a moving bed or a fluidised bed.

The elevated temperature may suitably be in the range from 50 to 300°C, though this will depend to some extent on the nature of the carboxylic acid reactant, the catalyst employed and the mode of operation. For monobasic acids the elevated temperature will generally be in the range from 100 to 300°C, preferably from 150 to 250°C. Using unsaturated dibasic acids the elevated temperature will generally be in the range from 50 to 350°C, preferably from 150 to 300°C. The pressure will generally be in the range from 1 to 300 barg, though again this will depend on the aforementioned factors.

Apart from the catalyst, the principal parameter influencing the selectivity to ester formation is the conversion. The conversion below which the desired selectivities to ester are achieved will depend on the nature of the carboxylic acid to be hydrogenated. Typically, for acetic acid hydrogenation, for example, it may be necessary to limited the conversion of acid to less than 90%. By operating at low conversions to achieve high selectivities to ester it is possible to recycle the unconverted acid and to reduce separation problems.

Catalyst Preparation

Example 1

An aqueous solution comprising 8.4036g palladium nitrate solution (7.63 wt% palladium, supplied by Johnson Matthey), 1.0107g of silver nitrate (supplied by Aldrich) and 17ml of deionised water was added to 25.00g of anatase titania (supplied by Norton sample no. 8965099). This was stirred for one hour before the addition of 2.60g of sodium disilicate (supplied by Fisons) in 120ml of deionised water. This mixture was stirred briefly then allowed to stand undisturbed for 24 hours. After this period the aqueous phase above the titania was treated with 10.00g of 85% hydrazine hydrate solution (supplied by Aldrich). The mixture was gently stirred and then allowed to stand undisturbed for 48 hours. The aqueous solution was then removed on a rotary evaporator and the resulting impregnated titania dried overnight at 125°C.

This catalyst was then mixed with an aqueous solution containing 1.9901g ammonium perrhenate (supplied by Johnson Matthey), the solvent again being removed on a rotary evaporator and the composition dried overnight at 125°C. This preparation produced a catalyst of nominal loading of 2.5%Pd-2.5%Ag-5%Re.

Comparative Catalyst Preparation 1

Example 1 was repeated except that silver nitrate was omitted from the preparation. This gave a catalyst of nominal loading of 2.5% Pd - 5% Re. This catalyst is not an example according to the present invention as it does not comprise an alloy of a Group VIII noble metal.

4

Catalyst Testing

A 500ml zirconium autoclave was purged with nitrogen and then charged with catalyst (2.0g) and appropriate solution. For acetic acid hydrogenation this consisted of 100ml of pure acetic acid (supplied by BP Chemicals). For maleic acid hydrogenation this consisted of 100ml 31.15wt% maleic acid in deionised water. This was prepared by dissolving the appropriate amount of maleic anhydride (supplied by Aldrich) in the solvent. The autoclave was then purged using hydrogen and then pressurised to 100 barg with hydrogen. Heating and stirring was commenced (5°C/min, 1000rpm) until 220°C was attained. This temperature was maintained for twelve hours with continued stirring. After this period the heating/stirring was ceased and the autoclave allowed to cool to room temperature. Gas and liquid phases were sampled and analysed by gas-liquid chromatography and high performance liquid chromatography. The results are shown in Tables 1 and 2. Nominal loading is defined as weight of metal (not metal compound) added to component (b) expressed as a percentage of the weight of the catalyst. Conversion is defined as the proportion of carboxylic acid/anhydride converted to products. Selectivity is defined as the proportion of reacted acid/anhydride which is converted to a particular product. Productivity is defined as weight of products per weight of catalyst per hour.

TABLE 1

| Acetic Acid Hydrogenation | | |
|---|---|---|
| | Comparative Catalyst 1 | Example 1 |
| | 2.5%Pd-5%Re/TiO$_2$ | 2.5%Pd-2.5%Ag-5%Re/TiO$_2$ |
| Product Selectivity(%) | | |
| Methane | 0.0 | 0.0 |
| Ethane | 0.0 | 0.0 |
| Ethanol | 11.9 | 10.1 |
| Ethylacetate | 88.1 | 89.9 |
| Conversion (%) | 26.7 | 22.7 |
| Productivity (kg/kgcat/hr) | 1.2 | 1.0 |

The results in Table 1 indicate that the silver alloyed catalyst is more selective to the ester (ethyl acetate) than the catalyst without the silver alloying component.

5

TABLE 2

| Maleic Acid Hydrogenation | | |
|---|---|---|
| | Comparative Catalyst 1 | Example 1 |
| | 2.5%Pd-5%Re/TiO$_2$ | 2.5%Pd-2.5%Ag-5%Re/TiO$_2$ |
| Product Selectivity(%) | | |
| Methane | 0.0 | 0.0 |
| Ethane | 0.0 | 0.0 |
| THF | 18.9 | 16.1 |
| Propanol | 0.2 | 0.2 |
| Butanol | 2.1 | 0.8 |
| GBL | 72.9 | 80.4 |
| BDO | 1.3 | 1.0 |
| Acetic Acid | 2.9 | 0.0 |
| Proprionic Acid | 0.3 | 0.0 |
| n-Butyric Acid | 1.4 | 1.5 |
| Selectivity to C$_4$ solvents (GBL,THF,BDO) | 93.1 | 97.5 |
| Conversion (%) | 48.4 | 43.4 |
| Productivity (kg/kgcat/hr) | 0.6 | 0.5 |

The results in Table 2 illustrate the benefit of the alloying component. Here it improves the selectivity to the internal ester, gamma-butyrolactone (GBL) as well as the combined selectivity to the valuable C$_4$ solvents:gamma-butyrolactone (GBL), tetrahydrofuran (THF) and 1,4-butanediol (BDO). By-product formation is also reduced.

**Claims**

1. A process for the production from a C$_2$ to C$_{12}$ carboxylic acid or an anhydride thereof of the corresponding carboxylic acid ester which process comprises reacting at elevated temperature the acid or anhydride with hydrogen in the presence as catalyst of a composition comprising (a) an alloy of (i) at least one noble metal of Group VIII of the Periodic Table of the Elements, and (ii) at least one metal capable of alloying with the aforesaid Group VIII noble metal, and (b) an oxide of an element of Group IVb of the Periodic Table of the Elements.

2. A process according to claim 1 in which component (b) comprises titania.

3. A process according to claim 2 in which the titania principally comprises anatase titania.

4. A process according to any one of the preceding claims in which the catalyst comprises for 0.1 to 20% of component (a).

5. A process according to any one of the preceding claims in which component (a) comprises an alloy of palladium, rhodium or ruthenium.

6. A process according to any one of claims 1 to 4 in which component (a) comprises an alloy of palladium with silver, gold, copper, nickel, rhodium, tin, cobalt, aluminium, mangnese, gallium, iron, chromium or platinum.

7. A process according to any one of the preceding claims in which the catalyst further comprises rhenium.

8. A process according to any one of the preceding claims in which the catalyst further comprises at least one metal selected from Groups IA or IIA of the Periodic Table of the Elements.

9. A process according to any one of the preceding claims in which the catalyst is supported on an inert support.

10. A process according to any one of the preceding claims in which the carboxylic acid comprises acetic acid, proprionic acid, butyric acids or heptanoic acids.

11. A process according to any one of claims 1 to 9 in which the carboxylic acid comprises maleic acid.

6

12. A catalyst for use in the process of claim 1 comprising (a) an alloy of (i) at least one noble metal of Group VIII of the Periodic Table of the Elements, and (ii) at least one metal capable of alloying with the aforesaid Group VIII noble metal, and (b) an oxide of an element of Group IV b of the Periodic Table of the Elements.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 553 761 (HOECHST)<br>* claims 1-3,7 * | 1,2,5,6 ,9,11, 12 | C 07 C 67/00<br>C 07 C 69/14<br>C 07 D 307/33<br>B 01 J 23/68 |
| A | DE-A-2 602 894 (GAF)<br>* claim 1 * | 1 | |
| A | US-A-2 072 861 (AMEND)<br>* page 1, column 1, line 26 * | 1,2 | |
| A | EP-A-0 164 922 (BP)<br>* claim 1 * | 1 | |
| D,A | GB-A-1 551 741 (HOECHST)<br>* page 2, line 59-62, line 97 * | 1,2,7 | |
| A | DE-A-2 456 995 (EXXON)<br>* page 4, paragraph 3; page 9, paragraph 2 * | 1,2,7 | |
| A | DE-A-2 715 666 (HOECHST)<br>* page 6, paragraphe 3; page 7, paragraphe 4 * | 1,2,7 | TECHNICAL FIELDS SEARCHED (Int. Cl.5) |
| A | PATENT ABSTRACTS OF JAPAN<br>vol. 12, no. 455 (C-548)(3302), 29 November 1988; & JP-A-63179846 (IDEMITSU KOSAN) 23.07.1988 | 1 | C 07 C 67/00<br>C 07 C 307/00<br>B 01 J 23/00 |

The present search report has been drawn up for all claims

| Place of search | Date of completion of the search | Examiner |
|---|---|---|
| BERLIN | 28-08-1990 | PROBERT C.L. |